# EUROPEAN PATENT APPLICATION

(11) **EP 4 564 367 A1**
(43) Date of publication of application: **04.06.2025**
(21) Application number: 23212492.5
(22) Date of filing: 28.11.2023
(51) Int. Cl.: G16H 40/63, G16H 50/30, G16H 20/40, G16H 40/20

(54) **DEVICE, SYSTEM AND METHOD FOR DETERMINING A PATIENT'S NEED LEVEL**

(71) Applicant: Koninklijke Philips N.V., 5656 AG Eindhoven (NL)
(72) Inventor: SPELT, Hanne Adriana Alijda, 5656AG Eindhoven (NL); NAUTS, Sanne, 5656AG Eindhoven (NL); VAN EE, Raymond, 5656AG Eindhoven (NL); KLAASSEN, Remy, 5656AG Eindhoven (NL)
(74) Representative: Philips Intellectual Property & Standards

(57) **Abstract**

The present invention relates to a device (2) and method for determining a patient's need level for training the patient scheduled to undergo a medical procedure. The device comprises an input unit (21) configured to obtain performance information indicating the performance of the patient in performing a training program configured to train and prepare the patient for the scheduled medical procedure, a processor (22) and an output unit (23) configured to output the determined patient's need level or information determined based on or related to the patient's need level. The processor is configured to determine the patient's physiological arousal during the performance of the training program based on the obtained performance information, and to determine the patient's need level for training based on the patient's physiological arousal during the performance of the training program.

## Description

### FIELD OF THE INVENTION

The present invention relates to a device, system and method for determining a patient's need level for training the patient scheduled to undergo a medical procedure. The medical procedure may generally be any medical imaging procedure (e.g. a medical scan), a medical treatment procedure (e.g. a radiotherapy procedure) or any other diagnostic or interventional procedure.

### BACKGROUND OF THE INVENTION

Magnetic Resonance Imaging (MRI) and Computerized Tomography (CT) are widely used diagnostic tools in pediatric care that provide doctors with high-quality diagnostic images. However, undergoing a scan (especially an MRI scan) can be stressful, requiring children to lie perfectly still for some time in the range of 10 (CT) to 60 (MRI) minutes in a narrow tunnel of a large device, while it makes odd noises. For healthcare staff, scanning children can provide challenges to their workflow and to the diagnostic quality of the scan. Consequently, anesthesia is used in many hospitals for pediatric scans. Comprehensive patient preparation can reduce the need for anesthesia but requires a substantial time investment from staff.

For this reason, many hospitals have created child-friendly scan rooms, e.g., by using colored lighting, changing the appearance of the scanner (making it look like a castle or submarine), or allowing children to watch a movie during the scan. Another common approach is to prepare and practice with patients in the imaging facility, for example with an educational video or face-to-face training using a mock scanner or simulated scanning sessions. Some hospitals offer solutions to prepare children at home, such as preparatory videos, brochures and hospital-specific mobile apps. Other imaging facilities combine preparation at home and in the hospital, sometimes in addition to adjustments within the scan rooms. Many of these approaches can improve the scan experience for pediatric patients, while increasing the rate of successful awake scans. It is thus possible to reduce sedation / anesthesia rates in pediatric medical imaging through preparation and guidance.

The Scan Buddy app (released by Philips in 2022) was developed to do that: prepare pediatric patients (particularly at the age of 4-8 years) and their caregivers for an MRI scan, in the days or weeks prior to the scan, in the comfort of their home. The iterative design process and a feasibility test of the Scan Buddy app are e.g. described in Saini, P., Koehn, C., Heuvelink, A., Tasar, O., van Vorstenbosch-Lynn, E., Nauts, S., & Trout, A. T. (2022, June). Scan Buddy: A Gamified App to Prepare Children for an MRI Scan. In Human-Computer Interaction. Theoretical Approaches and Design Methods: Thematic Area, HCI 2022, Held as Part of the 24th HCI International Conference, HCII 2022, Virtual Event, June 26-July 1, 2022, Proceedings, Part I (pp. 594-612). Cham: Springer International Publishing. The Scan Buddy app provides a learning framework based on informing, familiarizing, and training children for MRI and represents a gamified app with different learning goals.

The amount of training that a child needs for conducting, e.g., an awake scan is an important aspect for workflow arrangements. If children arrive at the hospital requiring additional preparation or training, this can provide challenges in terms of scheduling and workflow. In-hospital preparation and training require time and resources (e.g., sufficient time before the scan; availability of child life specialists or other hospital staff; availability of preparation resources such as a preparation room), that are not always available if children arrive at the hospital.

### SUMMARY OF THE INVENTION

It is an object of the present invention to provide a device, system and method for determining a patient's need level for training the patient scheduled to undergo a medical procedure, in particular to improve workflow and scheduling of medical procedures of patients.

In a first aspect of the present invention a device for determining a patient's need level for training the patient scheduled to undergo a medical procedure is presented, the device comprising:
- an input unit configured to obtain performance information indicating the performance of the patient in performing a training program configured to train and prepare the patient for the scheduled medical procedure;
   a processor configured to
- determine the patient's physiological arousal during the performance of the training program based on the obtained performance information, and
- determine the patient's need level for training based on the patient's physiological arousal during the performance of the training program;
   an output unit configured to output the determined patient's need level or information determined based on or related to the patient's need level.

In a further aspect of the present invention a system for training a patient scheduled to undergo a medical procedure is presented, the system comprising:
- one or more measurement elements configured to measure the patient's performance in the training;
- a device for determining a patient's need level for training the patient according to any one of the preceding claims; and
- a rendering unit configured to render visual and/or audible information according to the patient's need level.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to perform patient stratification, i.e., to determine the patient's need level, for training the patient scheduled to undergo a medical procedure. The patient is performing a training before the medical procedure, e.g. at home. The performance of the patient during the training is measured, wherein the performance may include one or more of a plurality of elements and is represented by performance information. From this performance information physiological arousal is determined, which is used to determine the patient's need level for training, i.e., to determine which type, amount, and duration (or other features) of further training the patient requires to optimally prepare for the scheduled medical procedure.

Physiological arousal generally refers to the state of the body when it is stimulated, aroused or activated. This is a fundamental component of the physiological system or response that occurs in response to stress, exercise, or numerous other mental and physical stimuli. Arousal may thus include an increased (faster) heart rate, perspiration, and rapid breathing.

The proposed solution is flexible and adaptive to individual patient needs and allows optimal preparation of patients, e.g. at home, for a medical procedure such as an MRI or CT scan, or another diagnostic procedure (such as ultrasound, X-ray, fluoroscopy, PET-CT, etc.) or interventional procedure (such as radiotherapy, proton therapy, IGT-procedures, dentistry, vaccination, blood collection, etc.) or any other medical procedure that may be perceived as scary by a patient. Generally, the present invention is applicable for any medical procedure where personal coaching (or, in other words, patient preparation) improves the outcome of the medical procedure. Preferred areas of application are preparation for any medical procedure that a child is scheduled to undergo, such as an awake scan.

Stratification of patient's (e.g., children's) need level prior to a hospital visit can help to address scheduling and workflow issues. This may lead to one or more advantages: No surprises at the medical apparatus used for the medical procedure such as a scanner, in particular with respect to very anxious persons (e.g., children); child life specialists often have limited time but can then devote their time to children who need the extra attention the most.

The present invention is particularly used for stratification of children who will undergo an upcoming MR scan. It may capitalize on the child's level of anxiety and/or mastery of learning objectives, and stratification can be done based on, e.g., one or more of measuring physiology during training (e.g., through usage of an app), learning goal completion, a dedicated survey, other game play data (e.g., measuring the number of errors children make in a game, the number of times they play a certain game, how much difficulty they have keeping still in a Lying Still game, etc.).

According to a preferred embodiment, the processor is configured to
- determine stress and/or anxiety level of the patient during the performance of the training based on the patient's physiological arousal and
- use the determined stress and/or anxiety level to determine the patient's need level for training.

For instance, in case of failure to accomplish learning goals during the training, it may be useful to know the stress and anxiety level of the patient in order to determine if and which further training or other preparation may be useful for the patient.

According to another embodiment, the processor is configured to
- determine, based on the patient's physiological arousal,
   the amount of effort it takes the patient to work on learning goals and/or tasks of the training program, and/or
   the stress of the patient in case a learning goal and/or task of the training program has not or insufficiently been achieved, and
- use the determined amount of effort and/or stress to determine the patient's need level for training.

Efforts and stress are further indicators signaling if the patient is sufficiently prepared for the upcoming medical procedure or may need further training or preparation.

In another embodiment, the processor is configured to
- determine one or more arousal features of the determined physiological arousal by comparing the determined physiological arousal to a reference physiological arousal, in particular to determine one or more of intensity, duration, moments in time and gradient of the determined physiological arousal, and
- use the one or more determined arousal features to determine the patient's need level for training.

In this way, stratification of patients can be made more precisely allowing a better understanding of the patient's need level for training.

The training program preferably includes one or more of a computer program, a mock examination simulating one or more aspects of the scheduled medical procedure, a survey, and a game. For instance, the training may be implemented in the form of an app running on a user device, such as a smartphone, tablet, laptop or computer. The app may implement a game or mock examination simulating the upcoming medical procedure to help, e.g., a child to learn and understand what will happen during the upcoming medical procedure and in which environment it will happen.

The processor may be configured to use the determined patient's need level to set or change one or more of the timing, type, priority, operation, sequence and location of the scheduled medical procedure. For instance, recommendations may be given with respect of the scheduled medical procedure for consideration by the medical staff to update anyone of the settings of the scheduled medical procedure. In other embodiments, the settings may be changed automatically.

In an embodiment, the processor is configured to set or change one or more of the timing, type, priority, operation, sequence and location of the scheduled medical procedure based additionally on one or more of the capacity of staff preparing the patient for the medical procedure, expected benefits of preparation, and the expected benefits of the medical procedure, in particular of an awake imaging scan. This may further improve the workflow, utilization and scheduling of patients, medical procedures and staff usage.

Hereby, the processor may be configured to determine the expected benefits of the medical procedure based on the patient's age and/or the expected number of follow-up medical procedures and/or type of follow-up medical procedures. For instance, younger children may be at higher risk of side effects from anesthesia than older patients, i.e., for younger children the benefits of an awake medical imaging scan are large so that large efforts in training should be made for younger children to prepare them for an awake medical imaging scan.

The processor may further be configured to determine the patient's need level for training and/or to set or change one or more of the timing, type, priority, operation, sequence and location of the scheduled medical procedure based additionally on
- procedural information comprising information about medical procedures including the scheduled medical procedure and/or
- patient information comprising information regarding the patient.

For instance, the learning goals are often dependent on scan type or the training program may be configured to mimic the real scan considering the scan type at home or a child care specialist may be recommended to support the child specific to scan type and the personal information regarding the child.

In another embodiment, the processor is configured to modify the training program by adapting one or more of difficulty level, prioritization, content, level of detail, style of presentation, semantics, words, sounds and environment based on the obtained performance information and/or the patient's need level, in particular based whether or not and/or to which extent one or more learning goals have been achieved. Thus, generally there are a plurality of options to modify the training program to further optimize the training.

In another embodiment, the output is configured to output the obtained performance information and/or rescheduling information indicating one or more changes of timing, type, priority, operation, sequence and location of the scheduled medical procedure. For instance, such information may be displayed on a monitor or communicated to clinical staff to check the information and take appropriate actions, such as order more training for the patient, modify the training, modify the schedule of the medical procedure, etc.

The expression "performance information" shall herein be understood in a broad sense. Generally, performance information may include information regarding one or more of
- breath holding and/or breathing exercises,
- remaining akinetic,
- being in a predetermined or the same posture,
- required time for solving a task,
- achieved difficulty level,
- coping with emotions including one or more of stress, fear, and anxiety,
- achievements of learning goals,
- physiological measurements including one or more of heart rate, respiration rate, blood pressure, and skin conductance,
- pupil diameter and/or patter of eye movements,
- current and/or predicted stress,
- time-stamped progress in the operation of the training program,
- psychological measurements,
- speech,
- sound,
- image and/or video data,
- mood and/or energy level during a guided mediation,
- performance of mindful activities and/or relaxation exercises,
- searching for information,
- number of repetitions of a task,
- number and/or type of errors on a task,
- the pressure used to push buttons,
- the accelerometry of the training device, and
- self-reported experiences.

Depending on the implementation and the available means for getting performance information, different pieces of such information may be used.

Apart from a device as explained above, the presented system includes one or more measurement elements configured to measure the patient's performance in the training and a rendering unit configured to render visual and/or audible information according to the patient's need level. The rendering unit may comprise a display configured to display visual information to the patient and/or a loudspeaker to output audible information to the patient. The one or more measurement elements may include multiple different elements, such as any kinds of vital signs sensors, camera, microphone, any kind of feedback from the training program, etc.

The system may be implemented in a computer or a game console or a handheld device, in particular a smartphone, laptop or tablet. Generally, any programmable device may be used for implementing or including the proposed system and device and for executing the proposed method.

### BRIEF DESCRIPTION OF THE DRAWINGS

These and other aspects of the invention will be apparent from and elucidated with reference to the embodiment(s) described hereinafter. In the following drawings:
Fig. 1 shows a schematic diagram of an embodiment of a system according to the present invention;
Fig. 2 shows a schematic diagram of an embodiment of a device according to the present invention;
Fig. 3 shows a schematic diagram of an embodiment of a method according to the present invention; and
Fig. 4 shows a schematic diagram of an implementation of a system according to the present invention in the form of a mobile phone.

### DETAILED DESCRIPTION OF EMBODIMENTS

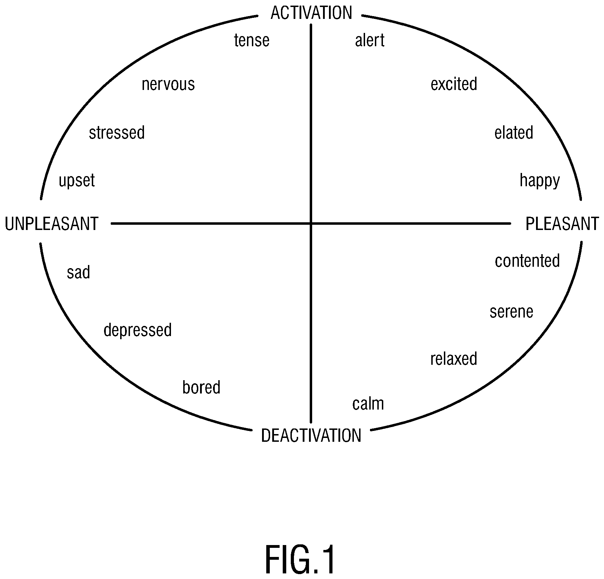

Psychological states characterized by negative valence and high arousal require intervention, such as nervous, tense, anxious, stressed. Fig. 1 shows a graphical representation of the circumplex model of affect with the horizontal axis representing the valence dimension and the vertical axis representing the arousal or activation dimension, as described in Posner, J., Russell, James A. & Peterson, Bradley S. (2005): "The circumplex model of affect: An integrative approach to affective neuroscience, cognitive development, and psychopathology", Dev Psychopathol, 17(3), 715-734. 10.1017/S0954579405050340. It is known from research that these psychological states and especially the activation level are reflected in physiological arousal, for example in the cardiovascular or electrodermal system, as shown in Table 1 below. Table 1 shows main functions, important measurable features and interpretations of activity changes of the cardiovascular, electrodermal, respiratory and facial psychophysiological systems, as described in Spelt, H. A. A. (2021), "Towards Physiology-Aware Persuasive Technology: A study on psychophysiological reactions to persuasive messages", PhD Thesis 1 (Research TU/e / Graduation TU/e), Industrial Engineering and Innovation Sciences, Eindhoven University of Technology. This means that - in addition to achieving the learning objectives - the arousal while working on the learning objectives can provide insight into the extent to which the child is ready for a scan/requires more training.

**Table 1**

| Physiological subsystem | Measurable features | Psychological meaning |
|---|---|---|
| *Cardiovascular system* is responsible for blood flow throughout the body. Its main functions are the supply of oxygen and disposal of waste. The system is under hormonal and nervous system control (Cacioppo et al., 2007). | *Heart rate (HR)* is measured as the number of beats per minute. Sympathetic and parasympathetic activity can increase and decrease HR, respectively (Camm et al., 1996). | HR increases in states with a higher arousal levels, for example joy, fear, or cognitive demands. It decelerates in passive emotions and resting states, such as affection, or contentment (Jänig, 2003; Kreibig, 2010; Looff et al., 2019). |
| | *Heart rate variability (HRV)* reflects the beat-to-beat variability in HR and thereby the interplay between the sympathetic and parasympathetic nervous systems. | HRV indicates adaptive emotion regulation in both pleasant and unpleasant emotions. Reduced HRV indicates emotional dysregulation, such as anxiety, stress, or depression (Jänig, 2003; Kreibig, 2010). |
| *Electrodermal system* involves sweat gland activity. The system is solely innervated by the sympathetic branch of the nervous system (Boucsein, 2012; Cacioppo et al., 2007). | *Skin conductance level (SCL)* is the tonic component of skin conductance. | Electrodermal activity can reflect affect, attentional reactions or effort. SCL elevates during experiences that call for action or evoke stress (Brouwer et al., 2018). |
| | *Skin conductance responses (SCRs)* are rapid phasic components. SCRs are measured as the number or magnitude of the skin conductance peaks. | SCRs can arise in response to a stimulus and their magnitude reflects emotional levels independent of the valence of the stimuli. The presence of SCRs can indicate reward focus or decision-making. |
| *Respiratory system* consists of all organs involved in breathing. Its primary task is oxygen supply and carbon dioxide depletion. Breathing can occur both automatically and intentionally (Cacioppo et al., 2007). | *Respiration rate (RR)* can be measured via mechanical movement of the diaphragm and rib muscles | Changes in RR relate to cognitive demands, for example high task difficulty or working memory load, as well as emotional processing, for example breathing rate is faster in disgust or sadness, slower in relief, and stops in surprise. |
| *Facial muscles* are skeletal muscles on the face and used to control conscious and unconscious facial expressions (Boxtel, 2010). | *Zygomaticus major (EMG-ZM)* activity is measured from the muscles located between the cheekbones and lip-corners. | *EMG-ZM activity* causes the lip-corners to go up. This is known as smiling and associated with psychological states of positive valence. |
| | *Corrugator supercilii (EMG-CS)* activity is measured from the muscles located at the medial end of the eyebrows. | *EMG-CS activity* causes frowning and associates with negative emotions, for example anger or sadness. Frowning also occurs with increased cognitive demands, for example reading or thinking |

In the following the present invention is largely illustrated using an example for the case when a child between 4 and 8 years is scheduled to undergo a scan such as an MRI examination. The present invention may, however, be adapted for other medical procedures and patients, such as children of other age groups or adults as well. In some cases alternatives are mentioned, but if this is not the case, then the example is not limiting the scope of the claims to exclude any other age groups or medical procedures.

In other words, any reference to a child shall be understood as generally meaning a patient. Any reference to a scan or exam shall be understood as generally referring to a medical procedure. Any reference to an app shall be understood as generally referring to a training program.

Having an MRI scan can make children anxious and prevent them from keeping still for a scan, and in general be less than cooperative. This has a negative impact on the diagnostic quality of the images and may increase the need for costly repeat scans. For this reason, many young children require sedation or general anesthesia (GA) to undergo a scan. However, using sedation/GA is costly: a scan with GA is approximately 9 times more expensive than an awake scan, in particular due to fewer required resources (no hospital bed, no anesthetic or personnel needed to provide GA, no additional time needed before a scan, and shorter waiting lists). There are studies that suggest that sedation/GA can have potentially adverse health effects. Preparing a patient for an awake scan can thus avoid unnecessary exposure to general anesthesia.

Digital tools such as smartphone applications (e.g., the Scan Buddy app) can provide hospitals with a scalable way to prepare patients. Digital tools can be used in combination with live, face-to-face preparation to augment it, or (in cases in which face-to-face training is unavailable), digital tools can be used instead of face-to-face preparation. However, existing digital applications are generic and one-size-fits-all, they are not tailored to an individual patient's specific imaging procedure and needs. As such, not all children receive the optimal preparation for their specific medical procedure.

Patients that are scheduled to undergo a medical procedure will understand what is going to happen and what is required from them better and/or more efficiently if the information is supplied in a more interactive and engaging manner, which requires the patient to perform actions that cause the patient to immediately experience the consequences. A patient will be better prepared and more familiar with the scheduled medical procedure if the information the patient receives relates directly to the scheduled procedure (improving so-called transfer of learning). Further, a personalized, tailored experience is most efficient to prepare the patient to the scheduled procedure. It is hence advantageous to know the patient's need level for training of the patient to adapt or personalize the training and/or to update the scheduling or other parameter(s) of the workflow related to the scheduled medical procedure.

Compared to a generic one-size-fits-all app, an app (or, more generally, a training device and method) that is tailored and personalized will do a better job of preparing patients for their medical procedure. It can prepare patients for exactly those things that are relevant to them. It can provide a personalized learning experience that prepares patients for exactly those things that they need to know/master to the extent that they need to master them.

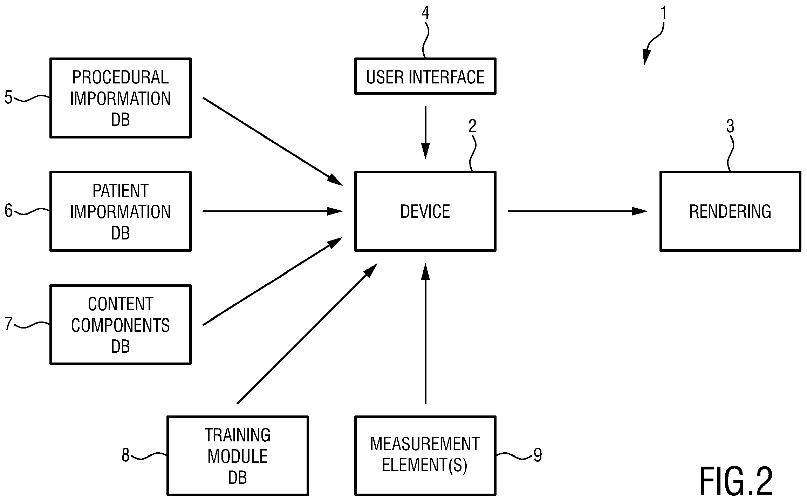

Fig. 2 shows schematic diagram of an embodiment of a system 1 for training a patient scheduled to undergo a medical procedure according to the present invention. The system comprises a device 2 for determining a patient's need level for training the patient as will be explained in more detail below and a rendering unit 3 configured to render visual and/or audible information according to the patient's need level, for instance according to control signals received from the device 2.

The system 1 further comprises one or more measurement elements 9 configured to measure the patient's performance in the training. These measurement elements 9 may e.g. comprise a processor that measures reactions of the patient performing the training (e.g. reaction time, kind of reaction, etc.), a video camera that monitors the patient performing the training, a score calculation means, vital signs sensors, etc.

The system 1 may be implemented in a computer or a game console or a handheld device, in particular a smartphone, laptop, tablet or computer.

The device 2 may generally be implemented by respective units or circuitry, e.g. a processor, processing circuitry, a computer, dedicated hardware, etc., that carries out the functions of the device. Alternatively, a common unit or circuitry, e.g. a common processor or computer, may implement the various functions of the device, or separate units or elements may be used that together represent the circuitry. In an exemplary implementation, a programmed processor may represent the device 2, which may execute a computer program that may be stored in a memory that is accessed by the processor.

The rendering unit 3 may generally be any means that outputs visual and/or audible information, e.g. in text form, as image or diagram, as sound or spoken words, etc. For instance, the rendering unit 3 may comprise (or be implemented as) a display configured to display visual information to the patient and/or a loudspeaker to output audible information to the patient. Exemplary implementation may use a touchscreen, a computer monitor, the screen of a smartphone or tablet, etc. as rendering unit 3.

The system 1 may further comprise a user interface 4 that is configured to receive user input, e.g. of procedural information and/or patient information. The user interface 4 may e.g. comprise a keyboard, touchscreen, microphone or any other entity that allows a user to enter information.

The system 1 may further comprise a procedural information database (DB) 5 containing procedural information comprising information about medical procedures including the scheduled medical procedure. The procedural information database 5 may be stored on a server, central storage of a hospital or medical service, in the cloud or at another location that is accessible by the device 2. The procedural information may e.g. be information about scan modality (such as MRI or CT), information about the body part to be scanned, scan sequences (duration; breath hold y/n; etc.), information whether or not contrast agent it required, planned orientation of the patient, etc.

The system 1 may further comprise a patient information database 6 containing patient information comprising information regarding the patient. The patient information database 6 may be stored on a server, central storage of a hospital or medical service, in the cloud or at another location that is accessible by the device 2.

The system 1 may further comprise a content components database 7 containing a plurality of content components for constructing a personalized training program, in particular comprising one or more of game components, video components, images, spoken and/or written text components, sound components, movie components for different medical procedures including the scheduled medical procedure, game asset modules, and educational modules. The content components database 7 may be stored on a server, central storage of a content provider, in the cloud or at another location that is accessible by the device 2. The content components database may be able to generate further content or provide the content to another entity for generating further content. For instance, the existing content may be used to generate new content, e.g. by applying predetermined content generation rules and/or methods, which may be part of the content components database as well.

The system 1 may further comprise a training module database 8 containing a plurality of training modules for constructing a training program. The training module database 8 may be stored on a server, central storage of a training module provider, in the cloud or at another location that is accessible by the device 2.

Two or more of the databases 5 to 8 may also be combined into common databases and/or stored at the same location, such as the cloud or archive or hospital's network.

The content components database 7 may be a database with a large variety of training module (also called app module) content components, preferably properly tagged with their content, to enable the construction of a personalized app experience. Examples of content components that may be part of the database are:
- various games, videos, images and spoken/written text that match specific learning goals (e.g., games that serve to teach children about "being familiarized with the sounds of the scanner", "understanding the importance of lying still", and/or "understanding that metal is not allowed into the MRI room");
- various scan sounds to personalize the digital intervention with the right (e.g., MRI or CT) sounds, and optionally the expected order of sounds;
- movies or movie clips for the various scan modalities, body parts, procedure steps to offer a movie embedding only correct and relevant information for the scheduled procedure;
- digital game assets such as models of MRI or CT scanners and scan accessories to enable displaying and interacting with these elements in a game catered to the type of modality and body part to be scanned; and
- educational materials to cater for scan modality and body part (e.g., education items to present to children during game play, information for parents/caregivers; frequently asked questions, etc.).

The training module database 8 may be database of generic training modules (also called 'app modules') in the form of interactive games with game play elements whose difficulty level or threshold for success can be adapted (e.g., the time or accuracy with which an object or body part has to be held still) and a method to tailor this difficulty level given the required level and the patient learning curve deduced from the game play data. To give an example, a lying still game in which children need to keep their phone still can have a low or high threshold for movement and it can differ in the time a patient needs to hold the phone still, etc.

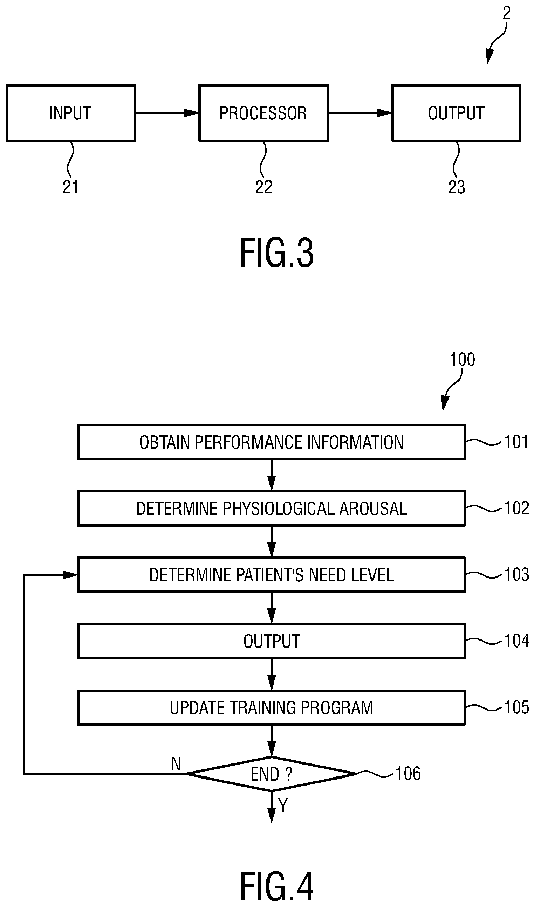

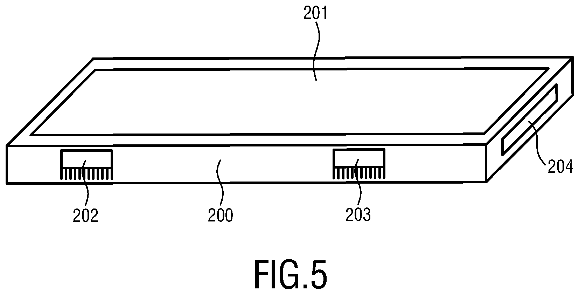

Fig. 3 shows a schematic diagram of a device 2 for determining a patient's need level for training the patient. The device 2 may further be configured for training a patient scheduled to undergo a medical procedure. The device 2 comprises an input unit 21 configured to obtain performance information indicating the performance of the patient in performing a training program configured to train and prepare the patient for the scheduled medical procedure, a processor 22 and an output unit 23 configured to output a determined patient's need level or information determined based on or related to the patient's need level to the rendering unit 3. The processor 22 is configured to determine the patient's physiological arousal during the performance of the training program based on the obtained performance information and to determine the patient's need level for training based on the patient's physiological arousal during the performance of the training program.

The input unit 21 may be directly coupled or connected to the user interface 4 or to the required one or more of the databases 5 to 8 or to one or more measurement elements 9 in order to obtain (i.e. retrieve or receive) the required information. The information may also be stored in a storage, buffer, network, or bus, etc. The input may thus e.g. be a (wired or wireless) communication interface or data interface, such as a Bluetooth interface, Wi-Fi interface, LAN interface, HDMI interface, direct cable connection, or any other suitable interface allowing information transfer to the device 2.

The processor 22 may be any kind of means configured to process the information and determine control signals there from. It may be implemented in software and/or hardware, e.g. as a programmed processor or computer or app on a user device such as a smartphone, smartwatch, tablet, laptop, PC, workstation, etc.

The output unit 23 may generally be any interface that provides the determined need level, e.g. transmits it to another device or provides it for retrieval by another device (e.g. a smartphone, computer, tablet, etc.). It may thus generally be any (wired or wireless) communication or data interface.

Fig. 4 shows a schematic diagram of an embodiment of a method 100 for determining a patient's need level for training the patient scheduled to undergo a medical procedure. The steps of the method 100 may be carried out by the device 2, wherein the main steps of the method 100 are carried out by the processor 22. The method 10 may e.g. be implemented as computer program running on a computer or processor.

In a first step 101 performance information is obtained that is indicating the performance of the patient in performing a training program configured to train and prepare the patient for the scheduled medical procedure. In a second step 102 the patient's physiological arousal during the performance of the training program is determined based on the obtained performance information. In a third step 103 the patient's need level for training is determined based on the patient's physiological arousal during the performance of the training program. In a fourth step 104 the determined patient's need level or information determined based on or related to the patient's need level is outputted.

Subsequently, in an optional fifth step 105, the training program is updated based on the patient's need level for training and, optionally, based on procedural information and/or patient information, and the updated training program may then be executed. For this purpose, control signals may be generated for controlling the rendering unit 3 to render visual and/or audible information according to execution of the training program. Steps 103 to 105 may be continuously / iteratively executed until an end criterion is reached, which may be checked in step 106. An end criterion may e.g. be that the user stops executing the training program or that a desired goal of the training program has been reached or that a time-out is reached.

Generally, the procedural information and/or the patient information can be generated through manual input (e.g. from staff), or it can be taken automatically from medical records (e.g., EMR systems), information systems, or other information sources. These information sources can be combined. The procedural information can be automatically pushed to the device, or it can be encoded in a link to an app running on the device 2 (e.g., it can be part of the information provided to patients/their caregivers). For example, patients may receive a (digital) invitation to the scan that includes a QR code containing information about the scheduled procedure.

Fig. 4 shows an exemplary implementation of a system 1 according to the present invention as a mobile phone (in particular as a smartphone) 200 having a display screen 201, an integrated processor 202, a motion sensor 203 and a loudspeaker 204.
The mobile phone 200 may be of any size, shape, model, etc., as long as it is suitable to handle the requirements of being used as a training system as disclosed herein.

The display screen 201 may be of any size or type (e.g. LCD, LED and the like). Screens with a large surface area and the capability of displaying strong colors or small and precise details are preferential. Preferably, the display 201 is integrated in the mobile device 200, but the visual content may also be duplicated or transmitted to an external display screen connected by wire or wirelessly to the rest of the handheld device, for instance a remote display screen, goggles or glasses, or any virtual reality device (e.g. virtual reality glasses). An integrated screen 201 is the most practical and compact solution, but an external screen, which by definition in the context of the presently claimed invention would still be part of the handheld device, even though that external screen may not be meant to be handheld or portable itself. Such an external screen may be easier to use in case the patient is not able to watch an integrated screen, e.g. due to the patient's position or other reason that prevents the patient from holding the handheld device and watching the integrating screen at the same time.

The processor 202 shall be capable of receiving, processing and analyzing incoming information and outputting visual and/or auditory information, in real time. The processor may also be in contact with a remote processor, for instance through the internet or through a wired or wireless, such as Bluetooth or Wi-Fi, connection with a local external processor, e.g. a workstation that may support the processor in processing power and/or may provide additional information, such as patient data and/or may store data of training sessions to be processed and/or reviewed automatically, by a physician for use in future sessions or to evaluate if the patient is sufficiently trained to undergo the medical procedure.

The motion sensor 203 shall be sensitive enough to detect relatively small and large motions caused by the user holding the handheld device 200. Most handheld devices are equipped with a motion sensor. These are usually relatively simple accelerometers, but more advanced accelerometers or other integrated motion sensors that would be suitable are possible as well.

It is preferable that the handheld device 200 is capable of providing auditory information, such as sound effects, music, coaching or other informative spoken information and the like to make the experience more immersive and effective. For this purpose, the mobile phone 201 is equipped with an integrated loudspeaker 204. Alternatively or simultaneously a headphone connection may be available to allow a headphone worn by the patient to be connected. The visual information, optionally augmented with auditory information, is preferably in the form of an (interactive) movie, a game or a manipulatable image. The visual information may be relatively simple, but also may be complex and rich in content.

The term `handheld device' shall be understood generally as covering any suitable device that is meant to be held by a user when in use, irrespective of whether the patient is actually holding the device or not. The handheld device is preferably a mobile phone as this is a device most people own or have direct access too. In other embodiments the handheld device may also be a tablet computer or a gaming device (e.g. a controller for a gaming computer or a mobile gaming computer).

The handheld device shall be able to detect information regarding the performance of the user during the training, e.g. to detect motion, reaction times, user operations, etc. Further, it shall be able to provide visual and/or auditory information to the user. Mobile phones, and particularly smartphones, which are standardly equipped with a display screen and a motion sensor are easily available, as are many types of tablet computers and gaming devices (although in those cases the display may be an externally connected display).

As most people own or have access to a suitable handheld device, they can be supplied with the software to run the training on their handheld device. If not, then they may be given a mobile device temporarily to use the training. An advantage is that the patient can do the training with the training device at a place and time which is convenient for them, for instance at home.

In the context of the present invention the term 'holding' should not be interpreted as that the patient is holding the handheld device only just by holding it using one or both of his or her hands, but also encompasses any other way in which the patient can control the balance of the handheld device. For instance, some patients may not be able to hold a device in one or both of their hands in a steady manner or the patient may not be able to hold the handheld device at all, but they might be able to lie still with their head, torso or other body part(s) that need to be still during the procedure. In those cases the patient may, for instance, 'hold' the handheld device by balancing it on other body parts, preferably those most relevant for the upcoming procedure, such as the torso (i.e. belly or back when lying down), knee, leg, arm, feet, head, etc., as long as the patient is able to influence mobility of the handheld device and receive feedback thereof.

In an embodiment, stratification of the patient may be done, i.e., the patient's need level may be determined, with learning goals and physiology. Vital signs of the patient may be measured while playing with an app implementing the training program to accomplish the learning goal. In case of failure to accomplish the learning goals the stress and anxiety level of the patient may be determined, e.g. from vital signs measurements, measurement of EDA (electrodermal activity), physiological signals, bodily signal (e.g., facial expression, pressure, etc.), self-reported stress, and others. Physiological arousal can be informative in at least two ways:
i) It reveals the amount of effort it takes a patient to work on the learning goals. In this case, the level of physiological arousal during a learning objective task may be compared with a baseline level. This baseline level is the physiological arousal of this person at rest. In case no baseline level is known, trend detection can be done. In case of stress an upward trend in sympathetic arousal may be expected; with no trend or a downward trend, the patient is okay. Alternatively, a comparison with a population average may be done.
ii) It reveals the stress it gives a patient when the app feeds back that they do something wrong. When someone receives feedback that he/she has not sufficiently completed the task, a short peak with instantaneous stress is expected. If the peak does not occur, or if the peak is not a peak but an entire increase in the sympathetic level, an intervention may be necessary.

The following measurements may be used: A camera may be used to detect the heart rate via skin color changes, pupil diameter, and/or patterns of eye movements. A wristband can measure skin conductance, estimate current stress and predict upcoming stress. Blood pressure can be measured by known PPG methods. In case of an app, time-stamped application data may reveal whether the patient is engaged in completing a learning goal. In case of other forms of preparation, time-stamped observations may be used to link the physiological trace to learning goal completion.

The stratification may then be based on the physiological arousal during these moments. Hereby, patients can be stratified in different ways, for example:
i. based on average physiology during app use. For most physiological measures, it is known which values are considered significant sympathetic activity.
ii. based on the increase (reactivity) in physiology compared to baseline or during a specific moment of app use. Higher reactivity is associated with higher arousal.
iii. if access to the patient database is available and used, a ranking can be made based on physiological arousal compared to other patients.

In another embodiment, stratification of the patient may be done with mock-exam and physiology. There may be an additional training in which the patient, e.g., a child, can experience the scan at home. This involves a mockup scan which mimics the ambient environment (sound and movie) from the real scan (also considering scan type). During the mock-scan, physiological aspects (or self-report) can be measured to assess user-state. It is also possible to measure specific learning goals, such as success at lying still.
This may be accomplished using a variety of means of doing a mock-exam, such as using a mockup scanner prior to the MRI appointment (e.g., life-size scanner or mockup; VR-experience of scan; small toy scanner such as Kitten scanner or Petit Prince, etc.), or through other data sources (e.g., data from a previous scans of the patient or similar kinds of patients, data from earlier play sessions, other preparation tools used including digital tools, multimedia tools, assessments from child life specialists, etc.).

By linking the mock-scan trace to the physiological trace it can be learned which elements of the scan make the user anxious (e.g., when a spike in arousal is seen) and/or how anxious users became from these elements (e.g., from the magnitude of the arousal). Repeating this procedure may provide insights as to whether anxiety levels as well as mastery of learning goals (such as children's ability to lie still) remain the same or deteriorate over time, indicating the need for additional training. Apart from that, the principle of stratification is the same as described above for the previous embodiment.

In another embodiment, stratification of the patient may be used for scheduling aid for staff members who need to prepare/guide pediatric patients (such as a Child Life Specialist, CLS). For instance, scheduling can be based on the amount of time the CLS has available (capacity), expected benefits of preparation (based on the stratification tool and data from previous scans), and the expected benefits of an awake scan. The benefits of an awake scan can be based on different criteria, such as the patient's age and clinical status (e.g., younger children may be at higher risk of side effects from anesthesia than older children), expected number of follow-up scans (children requiring many scans, e.g., in case of an oncology patient, may benefit more often from being able to undergo scans awake than children who only require a single scan), etc. As such, the stratification may be part of a larger scheduling tool for staff, that included the expected benefits of the CLS' time and prioritizes cases in which the relative benefit is highest (particularly in relationship to the required preparation time).

In another embodiment, stratification of the patient may be done by usage of app features. If children/patients often use app features that can be related to a certain aspect of the exam experience this may indicate where they need extra support. For example, tracking of mood and energy levels or doing guided mediation, breathing exercises, mindfulness activities or relaxation exercises may indicate that the patient is worried about the exam. Similarly, if someone is searching for a lot of information, he/she may not fully understand the exam to come.

In another embodiment, stratification of the patient may be done using a screening tool. For instance, a dedicated screening questionnaire may be included using, e.g., the Scan Buddy app. With the current Scan Buddy app, children can achieve several learning objectives in preparation for the scan. This screening tool can measure mastery of learning goals, as well as anxiety, scan experience, or other factors that may affect need for support.

For example, people that score high on personality traits such as neuroticism, perfectionism, low self-esteem, avoidance coping or overthinking/rumination can be associated with an increased tendency to worry or experience fear related to medical procedures. For example, someone who is high in neuroticism may be more likely to become anxious about medical procedures due to their heightened sensitivity to stress and threat. Someone who is highly perfectionistic may worry excessively about the potential outcomes of a medical procedure or may feel anxious about the idea of not being in control during the procedure. Similarly, someone with low self-esteem may worry about being judged by medical professionals or may be fearful of being seen as weak or vulnerable during the procedure. Those who use avoidance coping strategies may be more likely to delay or avoid necessary medical procedures, which can lead to increased worry or anxiety over time. Personality tests, such as NEO Personality Inventory, the Big Five Inventory, and the Eysenck Personality Questionnaire, can hint at anxiety levels.

This information may be shared upfront with the hospital, on which they may choose to schedule extra time or a particular CLS. In addition, in severe cases of failure to accomplish the learning goals it can be decided to re-schedule the upcoming scan date altogether.

In another implementation, patients may be prioritized based on this support need. Child life specialists often have limited time, so they can devote their time to children who need the extra attention the most. In addition, a screening tool may be used to schedule children for specific locations/time slots (e.g., if a child is likely to require training tools such as the Kitten Scanner, he/she should be scheduled for a location in which this tool is available).

In another embodiment, stratification of the patient may be used to pick a buddy, i.e., to select which character is the best buddy in the training program, e.g. the Scan Buddy app, for that specific patient. Some buddies are quite adventurous. Others are more empathic. Some buddies explain a lot, etc., other explain less. This may improve the patient's motivation to perform the training and achieve good results.

In another embodiment, stratification of the patient may be used for personalization. This embodiment concerns how to best address the patient in order to improve engagement. Based upon stratification a tone of voice may be automatically generated from the interactions of the patient with the app. In addition, recording conversations the patient has with care professionals can be used to e.g. extract the wording used by the patient and to check if a patient immediately understands instructions given or if multiple repetitions are needed. This may help to address the patient with the right wording (e.g., easy to understand, more sophisticated) and tone of voice (e.g., calming, casual, formal) so that instructions are understood, interaction is encouraged, and engagement is at a maximum. Patients may be stratified into personality or emotional profiles (e.g., drama queen, relaxed, anxious, etc.) so that the app response texts may be adapted to the patient to sooth that kind of person or personality and make them feel at ease.

In summary, the amount of training that a patient, such as a child, needs for conducting a medical procedure such as an awake scan is an important aspect for workflow arrangements. If patients arrive at the hospital requiring additional preparation or training, this can provide challenges in terms of scheduling and workflow. Such problems are addressed and prevented, or at least reduced, by the presented device, system and method that apply patient stratification, i.e., determine the patient's need level for training. Embodiments of this approach are based upon measuring user-state information, e.g., one or more of learning goal completion, sensor data, wearable physiological sensors, etc.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (2) for determining a patient's need level for training the patient scheduled to undergo a medical procedure, the device comprising:
an input unit (21) configured to obtain performance information indicating the performance of the patient in performing a training program configured to train and prepare the patient for the scheduled medical procedure;
a processor (22) configured to
determine the patient's physiological arousal during the performance of the training program based on the obtained performance information, and
determine the patient's need level for training based on the patient's physiological arousal during the performance of the training program;
an output unit (23) configured to output the determined patient's need level or information determined based on or related to the patient's need level.

2. Device according to claim 1,
wherein the processor is configured to
- determine stress and/or anxiety level of the patient during the performance of the training based on the patient's physiological arousal and
- use the determined stress and/or anxiety level to determine the patient's need level for training.

3. Device according to any preceding claim,
wherein the processor (22) is configured to
- determine, based on the patient's physiological arousal,
the amount of effort it takes the patient to work on learning goals and/or tasks of the training program, and/or
the stress of the patient in case a learning goal and/or task of the training program has not or insufficiently been achieved, and
- use the determined amount of effort and/or stress to determine the patient's need level for training.

4. Device according to any preceding claim,
wherein the processor (22) is configured to
- determine one or more arousal features of the determined physiological arousal by comparing the determined physiological arousal to a reference physiological arousal, in particular to determine one or more of intensity, duration, moments in time and gradient of the determined physiological arousal, and
- use the one or more determined arousal features to determine the patient's need level for training.

5. Device according to any preceding claim,
wherein the training program includes one or more of a computer program, a mock examination simulating one or more aspects of the scheduled medical procedure, a survey, and a game.

6. Device according to any preceding claim,
wherein the processor (22) is configured to use the determined patient's need level to set or change one or more of the timing, type, priority, operation, sequence and location of the scheduled medical procedure.

7. Device according to claim 6,
wherein the processor (22) is configured to set or change one or more of the timing, type, priority, operation, sequence and location of the scheduled medical procedure based additionally on one or more of the capacity of staff preparing the patient for the medical procedure, expected benefits of preparation, and the expected benefits of the medical procedure, in particular of an awake imaging scan, and/or
wherein the processor (22) is configured to determine the expected benefits of the medical procedure based on the patient's age and/or the expected number of follow-up medical procedures and/or type of follow-up medical procedures.

8. Device according to any preceding claim,
wherein the processor (22) is configured to determine the patient's need level for training and/or to set or change one or more of the timing, type, priority, operation, sequence and location of the scheduled medical procedure based additionally on
- procedural information comprising information about medical procedures including the scheduled medical procedure and/or
- patient information comprising information regarding the patient.

9. Device according to any preceding claim,
wherein the processor (22) is configured to modify the training program by adapting one or more of difficulty level, prioritization, content, level of detail, style of presentation, semantics, words, sounds and environment based on the obtained performance information and/or the patient's need level, in particular based whether or not and/or to which extent one or more learning goals have been achieved.

10. Device according to any preceding claim,
wherein the output (23) is configured to output the obtained performance information and/or rescheduling information indicating one or more changes of timing, type, priority, operation, sequence and location of the scheduled medical procedure.

11. Device according to any preceding claim,
wherein the processor (22) is configured to obtain, as performance information, information regarding one or more of
- breath holding and/or breathing exercises,
- remaining akinetic,
- being in a predetermined or the same posture,
- required time for solving a task,
- achieved difficulty level,
- coping with emotions including one or more of stress, fear, and anxiety,
- achievements of learning goals,
- physiological measurements including one or more of heart rate, respiration rate, blood pressure, and skin conductance,
- pupil diameter and/or patter of eye movements,
- current and/or predicted stress,
- time-stamped progress in the operation of the training program,
- psychological measurements,
- speech,
- sound,
- image and/or video data,
- mood and/or energy level during a guided mediation,
- performance of mindful activities and/or relaxation exercises,
- searching for information,
- number of repetitions of a task,
- number and/or type of errors on a task,
- the pressure used to push buttons,
- the accelerometry of the training device, and
- self-reported experiences.

12. System (1) for training a patient scheduled to undergo a medical procedure, the system comprising:
- one or more measurement elements (9) configured to measure the patient's performance in the training;
- a device (2) for determining a patient's need level for training the patient according to any one of the preceding claims; and
- a rendering unit (3) configured to render visual and/or audible information according to the patient's need level.

13. System according to claim 11 or 12,
wherein the system (1) is implemented in a computer or a game console or a handheld device, in particular a smartphone, laptop or tablet; and/or
wherein the rendering unit (3) comprises a display configured to display visual information to the patient and/or a loudspeaker to output audible information to the patient.

14. Method for determining a patient's need level for training the patient scheduled to undergo a medical procedure, the method comprising:
- obtaining performance information indicating the performance of the patient in performing a training program configured to train and prepare the patient for the scheduled medical procedure;
- determining the patient's physiological arousal during the performance of the training program based on the obtained performance information;
- determining the patient's need level for training based on the patient's physiological arousal during the performance of the training program; and
- outputting the determined patient's need level or information determined based on or related to the patient's need level.

15. Computer program comprising program code means for causing a computer to carry out the steps of the method as claimed in claim 14 when said computer program is carried out on the computer.
